# DEMANDE DE BREVET EUROPEEN

(11) **EP 0 673 628 A1**
(43) Date de publication de la demande: **27.09.1995**
(21) Numéro de dépôt: 95400579.9
(22) Date de dépôt: 15.03.1995
(51) Int. Cl.: A61F 5/453, A61F 5/24

(54) **Dispositif pénien permettant de pallier l'incontinence**

(30) Priorité: 17.03.1994 FR 9403363; 22.06.1994 FR 9407765
(71) Demandeur: Fondere, Jean Baptiste, F-34000 Montpellier (FR)
(72) Inventeur: Fondere, Jean Baptiste, F-34000 Montpellier (FR)
(74) Mandataire: Ravina, Bernard

(57) **Abrégé**

s La présente invention se rapporte à un dispositif (1) pour parer à l'incontinence constitué par un étui pénien souple (4) relié à une poche réceptrice (2) par un conduit (3), le dit étui (4) étant porté par une ceinture de taille (5) au moyen de pattes souples faisant partie intégrante de l'étui pénien.

## Description

L'objet de la présente invention se rapporte à un dispositif pénien permettant de pallier l'incontinence.

Il est connu dans ce domaine, de multiples dispositifs tels qu'une poche dans laquelle est introduite le pénis, où il se trouve dans un endroit confiné sans aération. De plus de tels dispositifs ne sont pas hygiéniques ce qui provoque chez les patients des irritations, brûlures et infections du pénis.

En effet, ce dernier ne pouvant être libéré dudit dispositif à tout moment, reste macéré toute la journée dans l'humidité urinaire.

Il existe également des dispositifs utilisant la colle ou tout autre adhésif afin de maintenirle pénis dans la poche d'évacuation, ce qui provoque bien souvent des irritations et ne permet pas à l'utilisateur d'en extraire son pénis pour en user normalement.

Il a été proposé également des dispositifs tels qu'énoncés dans le brevet UK 218471 ou dans le brevet US 4.994.051 qui comportent un bol rigide recevant à la fois le pénis et le scrotum.

Ce bol est fixé à la ceinture par sangles et comporte un conduit d'évacuation.

De tels dispositifs ne sont pas adaptés à un usage ambulatoire et sont encombrants.

La présente invention a pour objet de remédier à ces inconvénients en réalisant un dispositif peu encombrant et facile à utiliser en mode opératoire.

A cet effet, le dispositif permettant de pallier les incontinences faisant l'objet de l'invention est constitué d'un étui pénien souple relié à une poche réceptrice par un conduit, ledit étui étant maintenu à une ceinture de taille.

D'autres avantages et caractéristiques de l'invention apparaîtront à la lecture d'au moins un mode de réalisation qui sera mieux compris par l'intermédiaire des dessins annexés dans lesquels :
La figure 1 est une vue du dispositif selon l'invention.
La figure 2 est une vue du dispositif porté par un incontinent.
La figure 3 est une vue de profil d'un étui du dispositif selon l'invention.
La figure 4 est une vue de face du dit étui.
La figure 5 est une vue du dispositif pénien avec ceinture support.
La figure 6 est une vue du support du dispositif pénien.
Les figures 7 et 8 sont des vues d'une forme préférée de réalisation de l'étui pénien.

Le dispositif pénien 1 permet de pallier les problèmes d'incontinence. Ce dispositif comprend une poche 2 réceptrice d'urine reliée par un conduit 3, plus ou moins long selon l'emplacement de la poche, à un étui pénien. L'étui pénien occupe une position supérieure par rapport à celle de la poche et est destiné à recevoir le pénis ce qui permet de collecter l'urine qui peut alors s'écouler par gravité vers la poche 2 réceptrice, évitant ainsi au pénis de s'irriter ou encore de macérer et de risquer ainsi de s'infecter.

La forme de l'étui pénien est conique, facile d'accès, ce qui permet d'être utilisé pour différentes tailles de pénis. En partie inférieure cet étui présente un orifice par lequel il se connecte au conduit 3.

Ledit étui 4 est relié à une ceinture 5 fixée autour de la taille, de manière amovible par l'intermédiaire de boutons 6 ou encore de boutons-pressions ou des plots velcros maintenant ainsi l'étui pénien par au moins une patte de fixation 7.

L'étui pénien est de préférence réalisé en une matière synthétique non agressive pour la peau.

Avantageusement, il peut être réalisé en latex ou en caoutchouc synthétique.

Les pattes de fixation 7 sont partie intégrante de l'étui pénien et son souples de même que l'étui pénien.

Avantageusement, l'étui pénien est de dimension supérieure à celle du pénis qui y est libre à l'intérieur, ce qui évite toute pression sur celui-ci et toute agression sur la peau.

L'étui pénien selon l'invention est conçu comme un produit consommable qui peut être jeté quotidiennement.

Les boutons 6 ou les boutons pressions ou les plots velcros sont portés préférentiellement par deux ou plusieurs pattes 7 ménagées en partie supérieure de l'étui 4.

Ces pattes peuvent s'accrocher à la ceinture, soit de manière droite, soit de manière croisée pour accroître la stabilité et la tenue du pénis dans l'étui.

L'utilisateur, après avoir fixé sa ceinture 6 autour de la taille peut de part le mode de fixation amovible fixer l'étui pénien 4 après introduction dudit membre, et rejoindre l'emplacement de 2 de par le conduit souple 3.

Le mode de fixation de l'étui 4 à la ceinture 5 permettra à l'utilisateur d'uriner normalement après avoir dégraffé les pattes 7 reliant la ceinture 5 à l'étui 4.

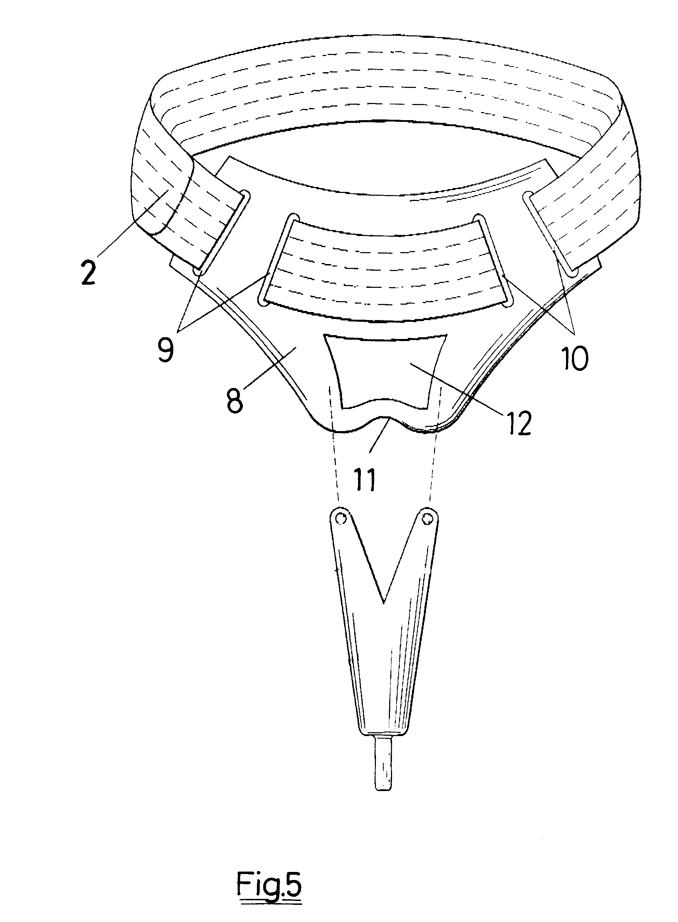

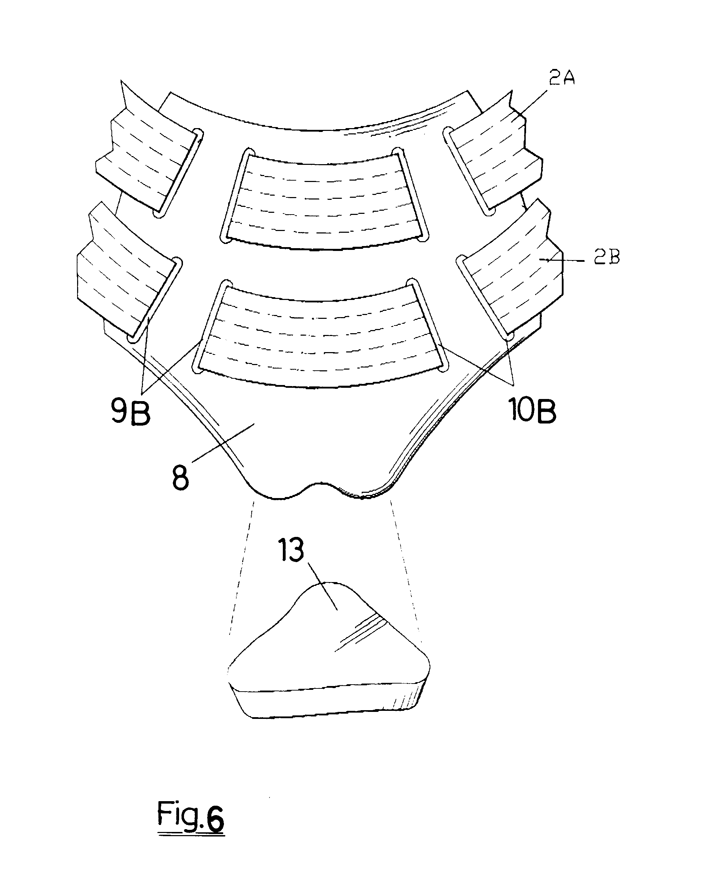

Aux figures 5 et 6 sont représentés des modes de réalisation de ceintures supports asociées à l'étui pénien.

Tel que représenté en figure 5, le dispositif selon l'invention comprend une cuirasse 8, une ceinture 2, un dispositif pénien 3.

La cuirasse 8 est de forme sensiblement triangulaire pour couvrir la partie ventrale jusqu'au dessus du pénis.

Elle est de forme galbée et est réalisée de préférence en un matériau rigide tel qu'une matière plastique de qualité adaptée.

Elle peut également être réalisée en un matériau semi-rigide ou du moins présentant une souplesse suffisante pour accepter un pliage (notamment en position assise de l'utilisateur) de son extrémité recevant le dispositif pénien.

La cuirasse est dotée de moyens de fixation de la ceinture 2.

Ces moyens permettent de fixer la ceinture en des positions diverses et variables de la cuirasse en sorte de faire varier la pression exercée sur la paroi ventrale.

Tels que représentés en figure 5, ces moyens sont constitués de mortaises réparties en deux groupes de deux mortaises parallèles 9 et 10, chacun des groupes étant disposé à un des angles de la cuirasse suivant une même génératrice de celle-ci.

La ceinture 2 peut être introduite, soit dans chacune des mortaises de chacun des groupes 9-10, soit dans une ou l'autre des mortaises de chacun des groupes 9-10, ce qui permet de faire varier la pression et la ou les zones de pression sur la paroi ventrale.

Il va de soi que sans sortirdu cadre de l'invention, la fixation de la ceinture et de la cuirasse peut être réalisée de toute autre manière connue.

La ceinture peut se fermer par une boucle ou par des plots auto-grippants.

Il faut noter que le mode préférentiel de fixation de la ceinture sur la cuirasse présente l'avantage, outre le faible coût de fabrication de la ceinture, de faciliter le centrage de la ceinture plus près du pénis de façon à mieux accrocher le dispositif pénien.

Avantageusement, l'extrémité inférieure 11 de la cuirasse qui vient au niveau du pénis présente une découpe, soit droite, soit comme représentée rentrante en arc de courbe.

La cuirasse est dotée de moyens de fixation du dispositif pénien.

De préférence, le moyen est constitué par un plot 12 auto-agrippant qui recevra un ou des plots complémentaires de la paroi arrière du dispositif pénien.

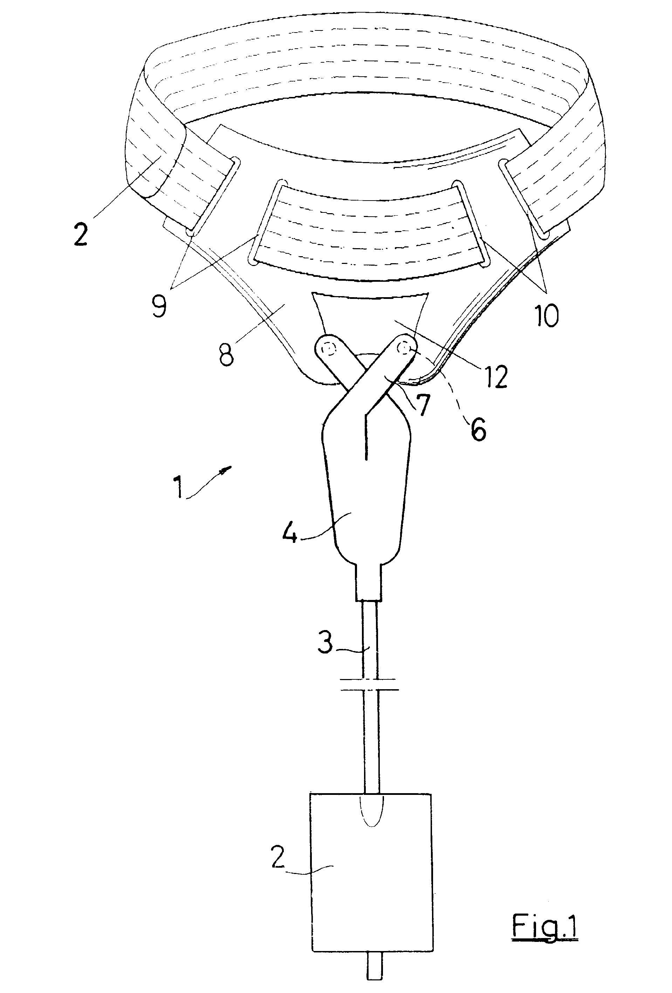

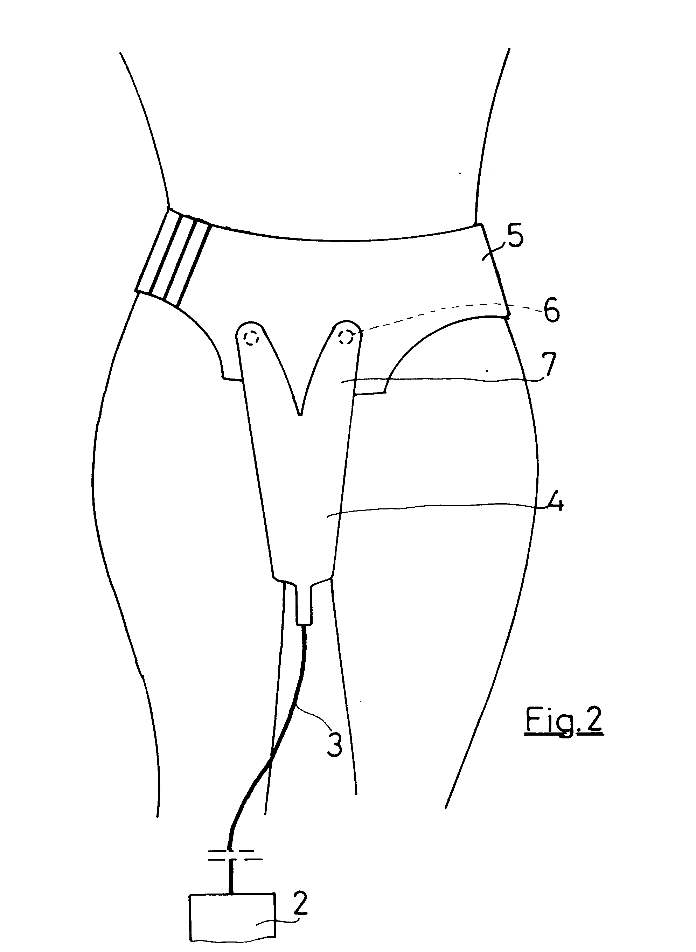

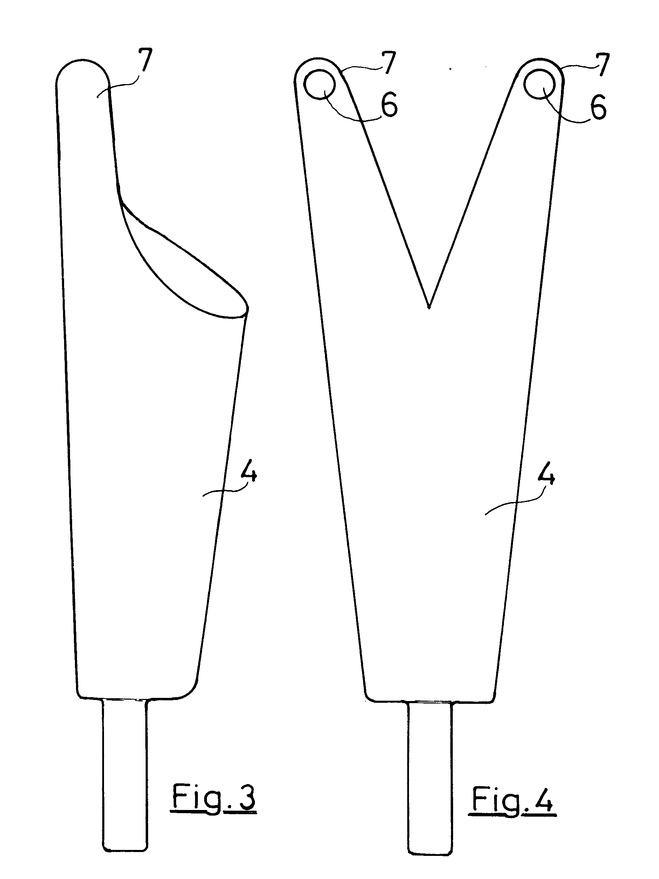

On peut également concevoir sans sortir du cadre de l'invention que le dispositif pénien soit fixé par tout autre moyen adapté tel qu'énoncé dans la description des figures 1 à 4.

En figure 6 est représentée une autre forme de réalisation de l'invention suivant laquelle la cuirasse 8 est dotée d'une ceinture en deux éléments 2A-2B qui coopèrent chacun avec deux groupes de deux mortaises 4A-5A et 4B-5B de la même manière que précédemment décrit, un groupe supérieur et un groupe inférieur.

Suivant les besoins ou l'anatomie du porteur, la cuirasse peut avoir de quatre à huit mortaises et recevoir une ou deux sangles ou ceinture.

La ou les sangles ou ceintures peuvent être élastiques ou non.

Selon l'invention, la cuirasse appuie sur la paroi ventrale de l'abdomen ; la pression ainsi répartie évite la descente d'organe, l'éventration ou la hernie.

Suivant une autre caractéristique de l'invention telle que représentée en figure 6, la paroi arrière de la cuirasse peut recevoir un ou plusieurs coussins 13 qui se placent entre elle et la paroi ventrale de l'utilisateur.

Avantageusement, le blocage en position du ou des coussins peut être obtenu par des plots auto- aggripants placés derrière la cuirasse à droite ou à gauche.

Suivant la morphologie de l'utilisateur, on peut accroître la pression au moyen d'une bande élastique passant d'avant en arrière sur la cuisse et fixée sur les plots auto-agrippants.

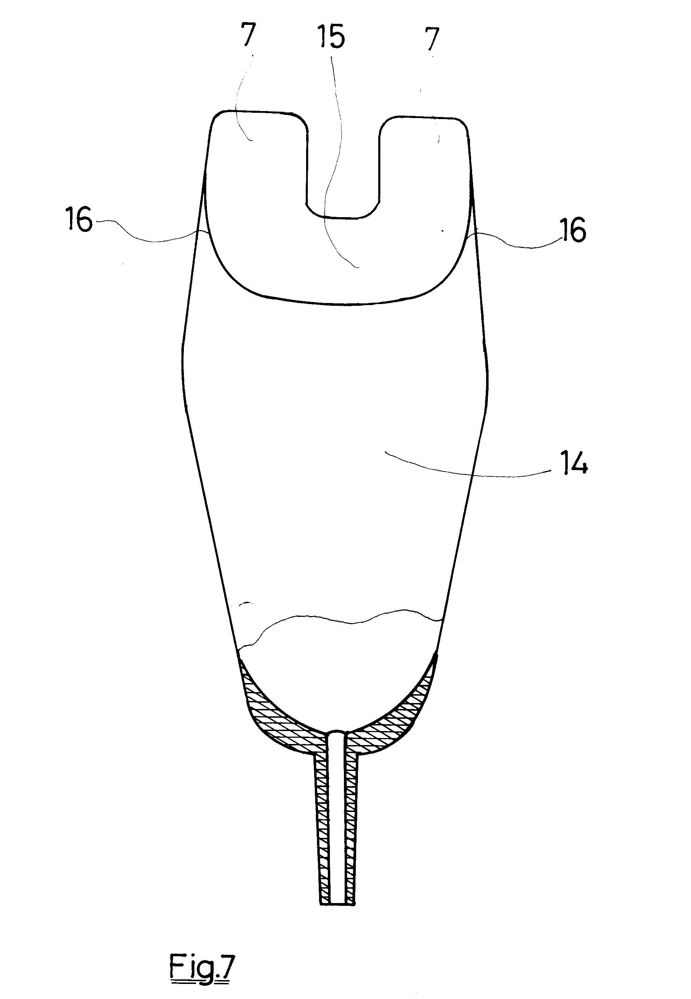

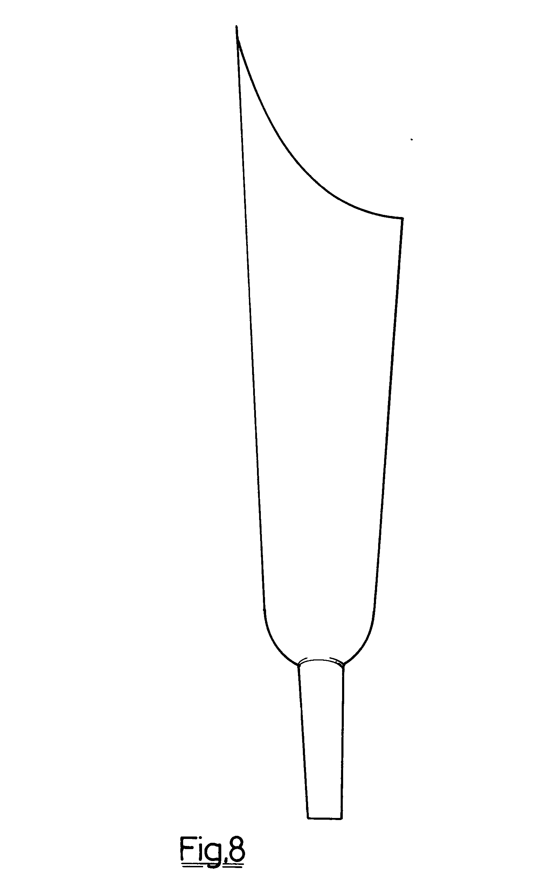

En figures 7 et 8 est représentée une forme préférentielle d'étui pénien.

L'étui selon cette forme de réalisation n'est pas de forme généralement cylindrique mais rectangle avec une face avant 14 et une face arrière 15 sensiblement aplaties et des côtés 16.

Selon cette forme de réalisation, l'étui pénien est réalisé en latex ou en caoutchouc synthétique.

Il se caractérise en ce que sa partie supérieure est très mince, ce qui est obtenable avec les matériaux employés et que la partie inférieure est plus épaisse notamment au niveau du tube de raccordement.

Les pattes 7 s'accrochent à la ceinture jointes et en parallèle pour faciliter l'aération du pénien en permanence.

## Revendications

1. Dispositif (1) pour parer à l'incontinence caractérisé en ce qu'il est constitué par un étui pénien (4) souple relié à une poche réceptrice (2) par un conduit (3), le dit étui (4) étant porté par une ceinture de taille (5) au moyen de pattes souples.

2. Dispositif selon la revendication 1 caractérisé en ce que l'étui (4) est accroché à la ceinture en parallèle et jointes, ce qui permet l'aération permanente du pénien sans laisser d'odeur ou croisé pour la position couchée pendant la nuit ; le scrotum se trouve ainsi libre.

3. Dispositif selon les revendications 1 et 2 caractérisé en ce que l'étui (5) pénien est fixé à la ceinture (5) par boutonnage.

4. Dispositif selon la revendication 3 caractérisé en ce que les boutons sont des boutons à pression ou normaux ou des plots velcros.

5. Dispositif selon la revendication 1 caractérisé en ce qu'il est doté d'au moins une patte (7) de fixation à la ceinture.

6. Dispositif selon la revendication 1 caractérisé en ce que deux pattes (7) espacées prolongent l'étui pénien dont elles font partie intégrante.

7. Dispositif selon la revendication 1 caractérisé en ce que l'étui pénien est conique.

8. Dispositif selon la revendication 1 caractérisé en ce que l'étui pénien présente une forme aplatie avec deux faces avant/arrière (14 et 15) sensiblement planes et deux côtés (16).

9. Dispositif selon la revendication 1 caractérisé en ce que l'étui pénien est de dimensions supérieures à celles du pénis.

10. Dispositif selon la revendication 1 caractérisé en ce que la paroi de l'étui pénien est mince.

11. Dispositifsupportd'un étui pénien selon la revendication 1 permettant de pallier l'incontinence caractérisé en ce qu'il est constitué d'une cuirasse ventrale (8) galbée, de forme sensiblement triangulaire, cette cuirasse étant dotée de moyens de fixation en diverses positions pour régler la pression sur la paroi ventrale d'au moins une ceinture (2) souple et de moyens de fixation d'un dispositif pénien (3).

12. Dispositif selon la revendication 1 caractérisé en ce que la cuirasse (8) est réalisée en un matériau rigide ou semi-rigide.

13. Dispositif selon la revendication 1 caractérisé en ce que la cuirasse (8) est dotée sur sa face arrière de moyens de fixation d'au moins un coussin (13) en des positions variables.

14. Dispositif selon la revendication 1 caractérisé en ce que les moyens de fixation de la ceinture sur la cuirasse sont constitués de mortaises (9-10) de passage de la ceinture.

15. Dispositif selon la revendication 14 caractérisé en ce que les moyens de fixation de la ceinture sur la cuirasse sont constitués de deux groupes de deux mortaises parallèles (9-10), chacun des groupes (9-10) étant disposé à un des angles de la cuirasse.

16. Dispositif selon la revendication 11 caractérisé en ce que l'extrémité inférieure de la cuirasse (8) est dotée d'une découpe (11) soit droite, soit en arc-de-courbe rentrant.
